# EUROPEAN PATENT SPECIFICATION

(11) **EP 0 668 927 B1**
(45) Date of publication and mention of the grant of the patent: **14.05.1997**
(21) Application number: 92903001.3
(22) Date of filing: 24.01.1992
(51) Int. Cl.: C12Q 1/68, C12N 15/10

(54) **ANALYSIS OF DNA**
DNS-ANALYSE
ANALYSE D'ADN

(30) Priority: 26.01.1991 GB 91017574
(43) Date of publication of application: 30.08.1995
(73) Proprietor: MEDICAL RESEARCH COUNCIL, London W1N 4AL (GB)
(72) Inventor: BROOKES, Anthony, Joseph 48 Hyde Avenue Stotfold, Hertfordshire SG5 4JD (GB); PORTEOUS, David, John Cherrytrees, Edinburgh EH4 9EH (GB)
(74) Representative: Matthews, Heather Clare
(86) International application number: GB9200144
(87) International publication number: WO9213100

(56) References cited:
- EP-A- 0 372 524
- WO-A-88/07585
- WO-A-89/01526
- WO-A-89/12695
- WO-A-91/03573

## Description

### Field of invention

This invention concerns analysis of DNA, in particular a method for recovering from two mixtures of DNA only those sequences present in both mixtures, i.e. coincident or shared DNA sequences in the two mixtures.

### Background to the invention

When analysing mixtures of DNA, particularly complex mixtures, it would be useful to be able to identify and recover those sequences present in two mixtures, and in recent years attempts have been made to achieve this end. Success so far has been very limited, although a few techniques have been developed which are applicable in limited, restricted situations. For example, a technique has been developed which enables isolation of human interalu fragments coincident between overlapping human-rodent somatic cell hybrids. WO-A-89/01526 discloses methods of obtaining common-sequence DNA fragments from two fragment mixtures. The present invention aims to provide a more versatile, generally applicable technique for isolating coincident sequences of DNA from two mixtures.

### Summary of the invention

According to the present invention there is provided a method of recovering from two mixtures of DNA those DNA sequences present in both mixtures, comprising treating DNA in a first mixture in a manner comprising the use of restriction enzyme(s) to produce single stranded DNA fragments with added defined flanking sequences flanking the single stranded DNA fragments; annealing capture oligonucleotides to the added defined flanking sequences of the resulting single stranded DNA; treating DNA in a second mixture using the same restriction enzyme(s) to produce single stranded DNA fragments; combining the products obtained from the first and second mixtures and allowing the single stranded DNA fragments to anneal; joining annealed single stranded DNA from the second mixture present as a heteroduplex to the capture oligonucleotides of DNA from the first mixture; and recovering from the resulting mixture sequences captured in the form of heteroduplex coincident DNA including the capture oligonucleotides.

Only DNA sequences present in both the first and second mixtures will form heteroduplex coincident DNA including the capture oligonucleotides, with a sequence from the first mixture carrying the capture oligonucleotides annealing with the coincident sequence from the second mixture. Other DNA fragments will either not anneal or will anneal with other single stranded DNA not including the capture oligonucleotides. The capture oligonucleotides can then be used to recover the heteroduplex DNA of interest, for example by using the polymerase chain reaction (PCR) against the capture oligonucleotides.

The method of the invention, like known methodologies, utilises the formation of heteroduplex species to distinguish between overlapping and non-overlapping components in two mixtures. However, the known methods utilise two double stranded mixtures of DNA in the formation of this heteroduplex and then achieve its isolation by some means dependent upon pretreatment of the ends of the DNA molecules. In contrast, in the method of the present invention the DNA from the first mixture is initially converted into a single stranded sequence with defined double stranded flanking sequences (by employing capture oligonucleotides). This structure can then act as a sequence specific trap for any related fragments in the unmodified second mixture. Once trapped a joining step, e.g. ligation, is then employed to join the ends of the trapped molecule to the capture oligonculeotides, i.e. specifically to tag the coincident sequences from the second source. By having the modified first source DNA in excess it is possible to drive heteroduplex formation to completion much more readily than is possible in alternative schemes.

Single stranded DNA fragments obtained from the first mixture by treatment with restriction enzymes are conveniently cloned into M13 (or other suitable cloning vehicles) to produce single stranded DNA copies with added defined flanking sequences (from M13 or the other suitable cloning vehicle) to which the capture oligonucleotides are annealed. Other techniques including PCR could also be used for this purpose. The use of PCR has the advantage that it allows for greater flexibility in the choice of the flanking sequences by altering the sequence of the PCR primers.

The capture oligonucleotides may be of any suitable length and are typically about 30-40 base pairs long, with for example about half of the sequence being designed to bind M13 and the other half being random.

The mixtures are conveniently treated with two restriction enzymes, e.g. EcoRI and PstI, but other combinations are clearly possible.

Depending on techniques used, it may be desirable to functionally isolate any unhybridised capture oligonucleotides before combining the products obtained from the first and second mixtures, and this is conveniently achieved by adding a further oligonucleotide with sequences complementary to the capture oligonucleotides. In particular, such treatment is desirable when PCR is used to recover the coincident DNA of interest.

As mentioned above, the sequences captured in the form of heteroduplex coincident DNA including the capture oligonucleotides may be recovered by PCR. Before using PCR it is necessary to separate these target sequences from the remainder of the first mixture DNA. This may be achieved by a size purification step, e.g. gel electrophoresis. Other techniques may alternatively be used, such as specific modification or degradation of the first mixture sequences achieved by virtue of their single stranded nature.

It may be possible to control the specificity of the method of regulating the degree of homology or identity required for recovery of coincident sequences. The method selects for DNA fragments which are perfectly matched at both ends but a degree of internal mismatch can be tolerated. However, it may be possible to control the stringency of the method, and by employing techniques such as single stranded modification and/or degradation of annealed heteroduplexes it may be possible for sequences which share up to 100% sequence identity to be recovered by the method of the invention.

The method of the invention is very versatile and generally applicable, and is useful in the analysis of complex mixtures of DNA. For example, the method may be used for isolating coincident sequences between complex sources such as total mammalian genome DNA, type A e.g. Human, and inter-mammalian somatic cell hybrid DNA, sub-AB e.g. a human chromosome or fragment thereof in a complete rodent genome, in the absence of a background of sequences derived from the rodent partner, or from human chromosome regions not represented in the hybrid cell. The method can also be used for isolating highly conserved DNA sequences between distinct species, and for isolating invarient DNA sequences between unrelated individuals from the same species, e.g. regions of linkage disequilibrium spanning disease genes in human populations. The method may also be used for integrating positional information with expression profile, i.e. for isolating candidate genes and exons based on combining available information on map location and tissue restricted expression. The method may also be applicable to microdissection libraries and cDNA libraries. It will be apparent that many other applications are also possible.

The method of the invention is thus of general interest and relevance to all genome mapping, evolution, genetic disease and expression studies.

The invention will be further described, by way of illustration, in the following Example and by reference to the accompanying figures, in which:
Figure 1 is a schematic representation of one embodiment of the method of the invention applied to two DNA mixtures A and B; and
Figure 2 is a Southern blot analysis of probes 3a and 4a.

### Example

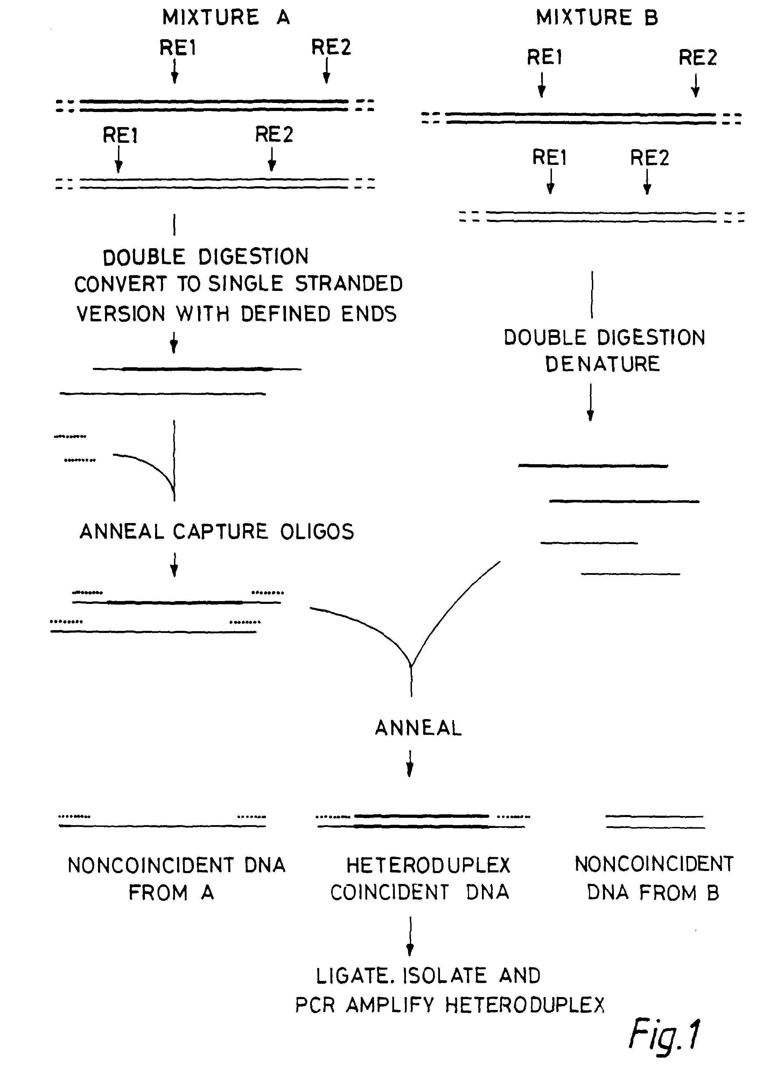

DNA sequences common to two mixtures of DNA, mixture A and mixture B, were recovered using the coincident sequence cloning (CSC) method illustrated schematically in Figure 1. Briefly, DNA fragments from mixture A were converted into short, orientated and single stranded molecules with added defined flanking sequences (from M13) at each end by first digesting with two restriction enzymes and then cloning into M13. A pair of synthetic capture oligonucleotides ('capture oligos') was then annealed to this modified form of mixture A (library A). Mixture B was digested with the same restriction enzyme pair that was previously used to process mixture A, and then alkali denatured. Library A and mixture B were combined and allowed to anneal in a reaction driven to completion by the components of library A. Following a ligation step, mixture B sequences were purified from those of library A by preparative alkali agarose gel electrophoresis. Coincident species were then selectively recovered from this material by employing the polymerase chain reaction with primers derived from the capture oligonucleotide sequences. PCR products were finally cloned and analysed.

In this example, DNA mixtures of high complexity were used, with mixture A comprising 1000 fragments of human DNA, and mixture B comprising the total DNA of a somatic cell hybrid called 1W1, which is a human-mouse hybrid with chromosomes 11 and Xpter as the sole human component (see reference 1).

To produce DNA mixture A total human DNA was digested to completion with the enzyme pair EcoRI/PstI and fragments of size 0.1-0.5kb were isolated by preparative agarose gel electrophoresis and cloned into EcoRI/PstI digested M13mp18. 1,000 plaques were picked at random and grown in 150µL cultures. These were pooled for the preparation of single stranded DNA. 5' and 3' capture oligonucleotides (see below) were added to 1µg of this DNA at a molar ratio of 1:1 in 40µL 10mM Tris/HCl, 1mM MgCl₂ pH7.5 and the mixture heated to 65°C and allowed to cool to 37°C over approximately 30 minutes. To bind any unhybridised capture oligonucleotides, oligonucleotide 485 (see below) was then added, in 1µL H₂O, at a molar ratio to each capture oligo of 10:1. This mixture was left at 37°C for 15 minutes and then placed on ice.

15µg of the DNA mixture B was digested with the enzyme pair EcoRI/PstI and then phenol, chloroform and ether extracted and ethanol precipitated. Following resuspension in 50µL H₂O, the sample was denatured by adding 50µL 0.34M NaOH and placing at 37°C for 30 minutes. 100µL of a prechilled 1:1 mixture of 0.34M HCl and 0.1M Tris/HCl pH7.5 was added to neutralise the solution and the sample placed on ice. The modified DNA from the first mixture was then added and the total sample ethanol precipitated. After resuspension in 18µL H₂O both 3µL of 4M NaCl, 50mM EDTA, 0.1M Tris/HCl pH7.8 and 9µL formamide were added and the mixture allowed to anneal by submerging overnight in a 45°C waterbath. The sample was precipitated and resuspended in 10µL 0.5M EDTA, 5mM Tris/HCl pH7.5. A ligation was then performed at 16°C for 3 hours in 20µL 50mM Tris/HCl, 10mM MgCl₂, 10mM dithiothreitol, 1mM spermidine and 1mM ATP pH7.4 using 0.5 units of T4 DNA ligase.

The ligated DNA was passed through a 1.3% preparative alkaline agarose electrophoretic gel (30mM NaOH, 2mM EDTA buffer) from which single stranded DNA fragments in the 0.1-0.5kb size range were recovered as set of five fractions called F1 - F5. The fractions were recovered as gel slices to be diluted twofold in H₂O and melted at 65°C.

The five fractions were each amplified by PCR using primers derived from the capture oligo sequences in order to recover coincident DNA. 30 cycles of PCR were performed upon 1µL aliquots of the purified DNA using oligos 596/789 (see below). 1µL of these reactions was then further amplified by 22 cycles of PCR using oligos 790/996 (see below).

All PCR reactions were carried out in 50µL PCR buffer (10mM Tris/HCl, 50 mM KCl, 1.5mM MgCl₂, 0.2mM each dATP/dTTP/dCTP/dGTP, 0.01%w/v gelatin, 0.05% each Tween20 and NP40 detergents, pH8.3 at 25°C) using 2 units Amplitaa enzyme and a Hybaid Intelligent Heating Block on mode 2 (plate) control. Denaturing steps were at 99°C for 45 seconds with an extended time of 2 minutes for the first cycle. Extension reactions were done at 74°C for durations of 2 minutes for the first ten cycles, 2.5 minutes for the second ten cycles and 3 minutes for any further cycles. Annealing steps were of 2 minute duration at 58°C for oligos 596/789 and 52°C for oligos 790/996.

The PCR products were separated on a 1.3% neutral agarose gel. Major bands were excised and recovered by agarose digestion, phenol/chlorform extraction and ethanol precipitation. These products were then cloned following EcoRI/PstI digestion into the pBluscribe plasmid vector, and plated to give product libraries F1 - F5.

To analyse the products of this experiment between 2 and 6 clones were picked at random from each product library. These were examined by cross-bybridisation. In the case of product library F3 two distinct isolates were thus obtained. These were used as probes upon library F3 enabling 14 non-hybridising colonies to be located. From there a further 3 distinct products were subsequently identified. These results are summarised in Table 1.

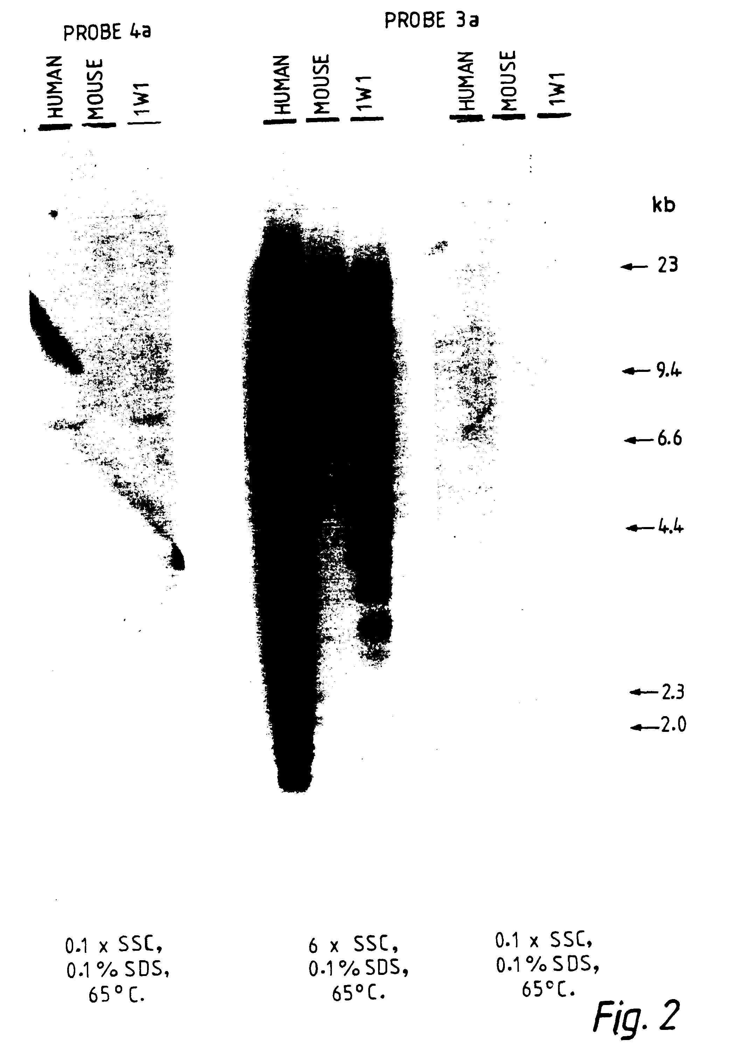

All distinct products were used to probe Southern blots of EcoRI digested human, mouse and 1W1 DNA in the absence of competitor DNA. In all cases the probes had clearly been derived from human sequences present within 1W1. In the majoriy of cases a single hybridising band was detected with only 1 case showing hybridisation to a high copy number repeat element. By sequencing each product it was observed that a number of isolates shared a similar sequence even though all had given unique single bands upon Southern analysis. One such product was therefore used as a probe on genomic blots under various stringencies. This family of products were thus shown to have been derived from a low copy repeat element. Sequence database searches were also performed for each coincident sequence cloning product. A summary of these results is presented in Table 2, in which "Line 1" is human KpnI repeat (reference 2). "L1HEG" is a sequence within a repeat rich region at the beta-globin cluster (reference 3), and "P450c17/SigmaG3" is a sequence upstream of both P450c17 (reference 4) and immunoglobulin heavy C gamma 3 (reference 5) genes. Examples of Southern blot results are shown in Figure 2. Washing stringencies for each experiment are given in the Figure.

All of the products obtained were foun to be genuinely coincident indicating a specificity of 100%. Furthermore given the proportion of the human genome present within 1W1 (5%) and the number of mixture A molecules examined (1,000) then the eight products isolated indicate a minimum recovery efficiency of 16%. The spectrum of the coincident species obtained probably reflects their abundance and/or ease of amplification by PCR.

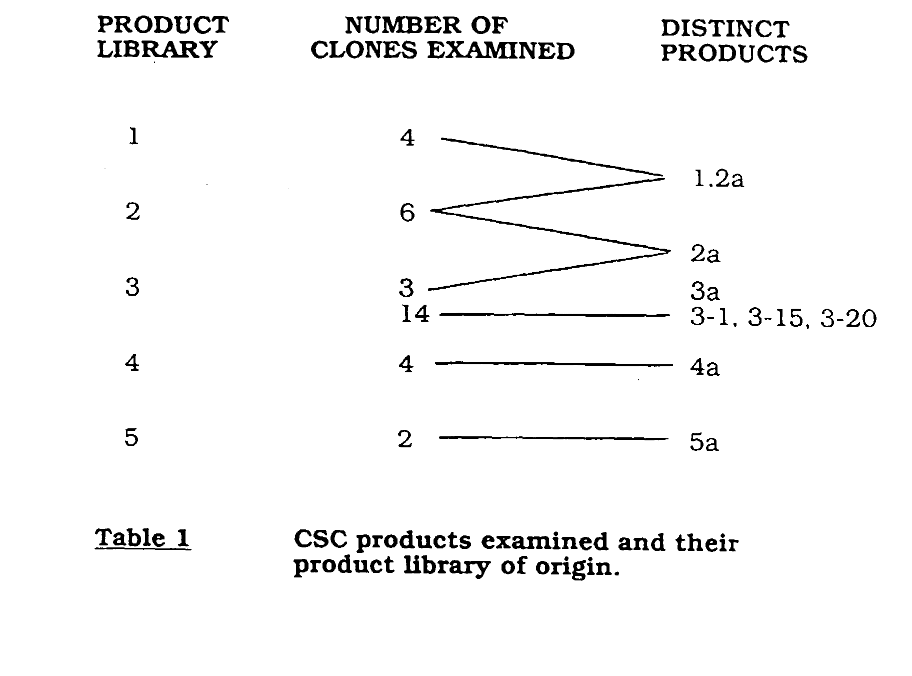

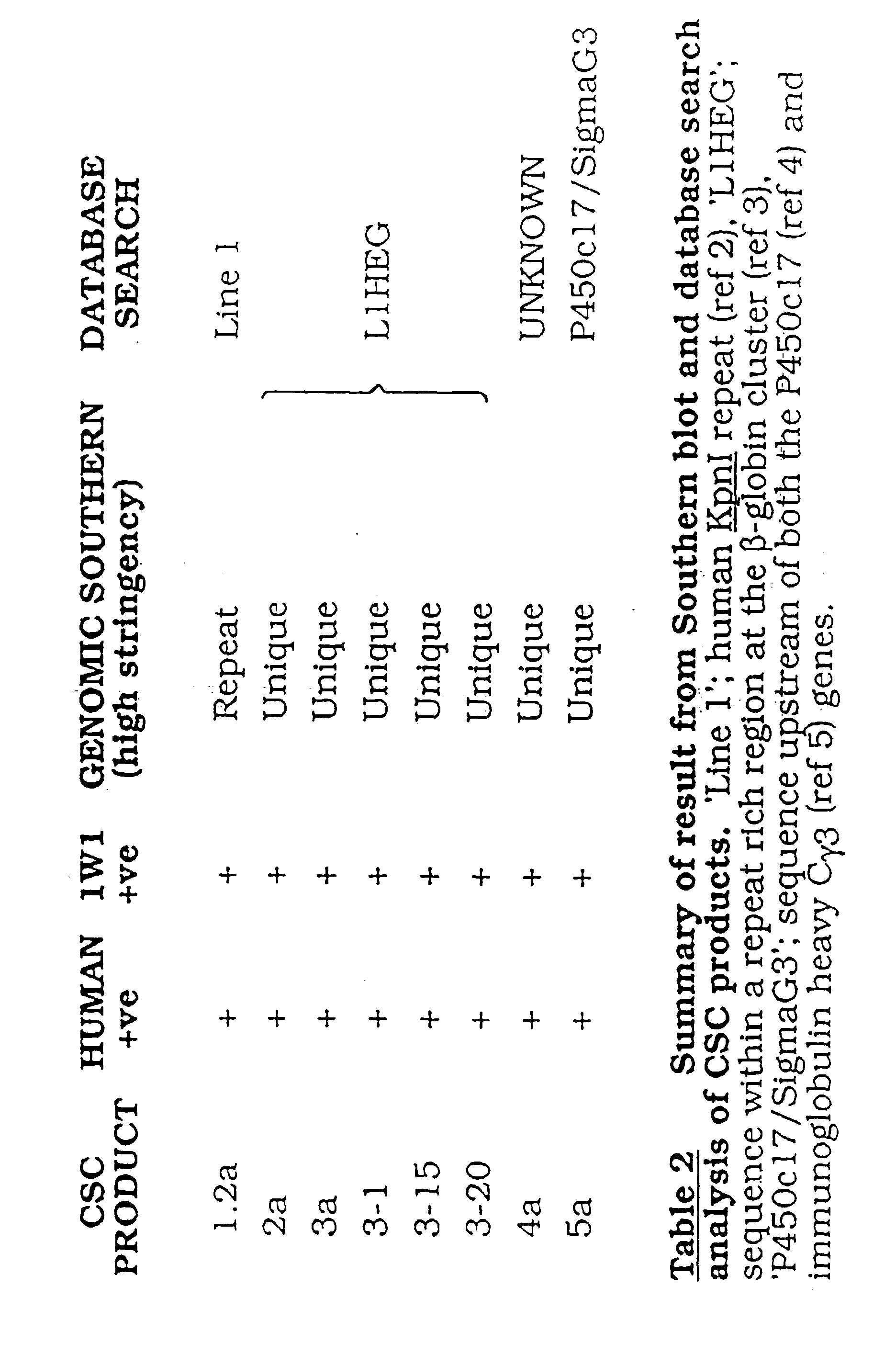

Oligonucleotide sequences

5' capture oligo:
GGACGGGTCGACACGCGAGGAGCCAAGCTTGCATGCCTGCA

3' capture oligo:
AATTCGTAATCATGGTCATAGAGCACCCGTGCTACCGGAACG

485:
TGATTACGAATTGGTGCAGGCATGCAAG

PCR oligos:
596 GGACGGGTCGACACGCGAGG
789 CGTTCCGGTAGCACGGG
790 GCCAAGCTTGCATGCCTG
996 GCTCTATGACCATGATTACG

### REFERENCES

1. Porteous, D.J., Morten, J.E.N., Cranston, G., Fletcher, J.M., Mitchel, A., vanHeyningen, V., Fantes, J.A., Boyd. P.A. and Hastie, N.D. (1986) Mol. Cell. Biol. 6, 2223-2232.
2. Skowronski, J., Fanning, T.G., and Singer, M. (1988) Mol. Cell. Biol. 8(4), 1385-1397.
3. Rogan, P.K., Pan, J. and Weissman, S.M. (1987) Mol. Biol. Evol. 4(4), 327-342.
4. Picado-Leonard, J. and Miller, W.L. (1987) DNA. 6(5), 439-448.
5. Hisajima, H., Nishida, Y., Nakai, S., Takahashi, N., Ueda, S. and Honjo, T. (1983) Proc. Natl. Acad. Sci. USA. 80, 2995-2999.

## Claims

1. A method of recovering from two mixtures of DNA those sequences present in both mixtures, comprising: treating DNA in a first mixture in a manner comprising the use of restriction enzyme(s) to produce single stranded DNA fragments with added defined flanking sequences flanking the single stranded DNA fragments; annealing capture oligonucleotides to the added defined flanking sequences of the resulting single stranded DNA; treating DNA in a second mixture using the same restriction enzyme(s) to produce single stranded fragments; combining the products obtained from the first and second mixtures and allowing the single stranded fragments to anneal; joining annealed single stranded DNA from the second mixture present as a heteroduplex to the capture oligonucleotides of DNA from the first mixture; and recovering from the resulting mixture sequences captured in the form of heteroduplex coincident DNA including the capture oligonucleotides.

2. A method according to claim 1, wherein DNA from the first mixture is produced in single stranded form by cloning in M13.

3. A method according to claim 1, wherein DNA from the first mixture is produced in single stranded form by PCR.

4. A method according to any one of the preceding claims, wherein the capture oligonucleotides are in the range of 30-40 bases long.

5. A method according to any one of the preceding claims, wherein an oligonucleotide complementary to the capture oligonucleotides is added to the products of the first DNA mixture before combining the products of the first and second DNA mixtures.

6. A method according to any one of the preceding claims, wherein heteroduplex coincident DNA is recovered from a mixture by means of PCR.

7. A method according to any one of the preceding claims, wherein the products of the second DNA mixture possess 100% sequence hormology with the products of the first DNA mixture to which they anneal.

8. A method according to any one of the preceding claims, wherein the coincident sequence identified by the method is a DNA sequence which is highly conserved between distinct species.

9. A method according to any one of claims 1 to 7, wherein the coincident sequence identified by the method is a DNA sequence which is invariant between unrelated individuals of the same species.

## Patentansprüche

1. Verfahren zur Isolierung jener Sequenzen aus zwei Gemischen von DNA, die in beiden Gemischen vorliegen, bei dem man die DNA in einem ersten Gemisch in einer die Verwendung von Restriktionsenzym(en) umfassenden Weise behandelt, um einzelsträngige DNA-Fragmente mit angefügten definierten flankierenden Sequenzen herzustellen, die die einzelsträngigen DNA-Fragmente flankieren; Abfang-Oligonukleotide an die angefügten definierten flankierenden Sequenzen der erhaltenen einzelsträngigen DNA hybridisieren läßt; die DNA in einer zweiten Mischung unter Verwendung des (der) gleichen Restriktionsenzyms(e) behandelt, um einzelsträngige Fragmente herzustellen; die aus der ersten und zweiten Mischung erhaltenen Produkte vereinigt und die einzelsträngigen Fragmente hybridisieren läßt; die als Heteroduplex vorliegende hybridisierte einzelsträngige DNA aus der zweiten Mischung mit den Abfang-Nukleotiden der DNA aus der ersten Mischung verbindet; und die in Form eines übereinstimmenden DNA-Heteroduplex gefangenen Sequenzen, einschließend der Abfang-Oligonukleotide, aus dem resultierenden Gemisch isoliert.

2. Verfahren nach Anspruch 1, bei dem die DNA aus der ersten Mischung durch Klonieren in M13 in einzelsträngiger Form hergestellt wird.

3. Verfahren nach Anspruch 1, bei dem DNA aus der ersten Mischung in einzelsträngiger Form durch PCR hergestellt wird.

4. Verfahren nach irgendeinem der vorstehenden Ansprüche, bei dem die Abfang-Oligonukleotide zwischen 30 und 40 Basen lang sind.

5. Verfahren nach irgendeinem der vorstehenden Absprüche, bei dem ein zu den Abfang-Oligonukleotiden komplementäres Oligonukleotid zu den Produkten des ersten DNA-Gemischs zugegeben wird, bevor die Produkte des ersten und zweiten DNA-Gemischs vereinigt werden.

6. Verfahren nach irgendeinem der vorstehenden Ansprüche, bei dem der übereinstimmende DNA-Heteroduplex durch PCR aus einem Gemisch isoliert wird.

7. Verfahren nach irgendeinem der vorstehenden Ansprüche, bei dem die Produkte des zweiten DNA-Gemischs 100%ige Sequenzhomologie mit den Produkten des ersten DNA-Gemischs, mit denen sie hybridisieren, aufweisen.

8. Verfahren nach irgendeinem der vorstehenden Ansprüche, bei dem die durch das Verfahren identifizierte übereinstimmende Sequenz eine DNA-Sequenz ist, die zwischen unterschiedlichen Spezies hochkonserviert ist.

9. Verfahren nach irgendeinem der Ansprüche 1 bis 7, bei dem die durch das Verfahren identifizierte übereinstimmende Sequenz eine DNA-Sequenz ist, die zwischen nicht verwandten Individuen derselben Art invariant ist.

## Revendications

1. Procédé de récupération, à partir de deux mélanges, d'ADN dont les séquences sont présentes dans les deux mélanges, comportant les étapes consistant à : traiter l'ADN d'un premier mélange d'une manière comportant l'utilisation d'enzyme ou d'enzymes de restriction pour produire des fragments d'ADN simple-brin munis de séquences d'encadrement ajoutées, déterminées encadrant les fragments d'ADN simple-brin ; hybrider des oligonucléotides de capture aux séquences d'encadrement ajoutées, déterminées de l'ADN simple-brin résultant ; traiter l'ADN d'un second mélange en utilisant la ou les mêmes enzymes de restriction pour produire des fragments simple-brin ; combiner les produits obtenus à partir du premier et du second mélange et permettre aux fragments simple-brin de s'hybrider ; ligaturer l'ADN simple-brin hybridé du second mélange, présent sous forme d'hétéroduplex, aux oligonucléotides de capture de l'ADN du premier mélange ; et récupérer à partir du mélange résultant les séquences capturées sous la forme d'ADN hétéroduplex coïncident portant les oligonucléotides de capture.

2. Procédé selon la revendication 1, dans lequel l'ADN du premier mélange est produit sous une forme simple-brin par clonage dans M13.

3. Procédé selon la revendication 1, dans lequel l'ADN du premier mélange est produit sous une forme simple-brin par PCR.

4. Procédé selon l'une quelconque des revendications précédentes, dans lequel les oligonucléotides de capture ont une longueur comprise entre 30 et 40 bases.

5. Procédé selon l'une quelconque des revendications précédentes, dans lequel on ajoute un oligonucléotide complémentaire des oligonucléotides de capture aux produits du premier mélange d'ADN avant de combiner les produits du premier et du second mélange d'ADN.

6. Procédé selon l'une quelconque des revendications précédentes, dans lequel un ADN hétéroduplex coïncident est récupéré à partir d'un mélange à l'aide d'une PCR.

7. Procédé selon l'une quelconque des revendications précédentes, dans lequel les produits du second mélange d'ADN possèdent 100 % d'homologie de séquence avec les produits du premier mélange d'ADN auxquels ils s'hybrident.

8. Procédé selon l'une quelconque des revendications précédentes, dans lequel la séquence coïncidente identifiée par le procédé est une séquence d'ADN qui est fortement conservée entre des espèces distinctes.

9. Procédé selon l'une quelconque des revendications 1 à 7, dans lequel la séquence coïncidente identifiée par le procédé est une séquence d'ADN qui est invariante entre des individus de la même espèce, non-apparentés.
